# EUROPÄISCHE PATENTSCHRIFT

(11) **EP 3 049 422 B1**
(45) Veröffentlichungstag und Bekanntmachung des Hinweises auf die Patenterteilung: **31.07.2019**
(21) Anmeldenummer: 14799951.0
(22) Anmeldetag: 26.09.2014
(51) Int. Cl.: C07F 9/54, C07D 233/74, C07D 233/76, C07D 233/88, C09D 5/18

(54) **INTUMESZENZBESCHICHTUNGEN**
INTUMESCENT COATINGS
REVÊTEMENTS INTUMESCENTS

(30) Priorität: 26.09.2013 AT 506222013
(43) Veröffentlichungstag der Anmeldung: 03.08.2016
(73) Patentinhaber: MIPA SE, 84051 Essenbach (DE)
(72) Erfinder: SALCHNER, Robert, 6020 Innsbruck (AT); SCHOTTENBERGER, Herwig, 6082 Patsch (AT); GRIESSER, Ulrich, 6094 Axams (AT); LACKNER, Roman, 6020 Innsbruck (AT)
(74) Vertreter: Müller, Christian Stefan Gerd
(86) Internationale Anmeldenummer: PCT/AT2014/050221
(87) Internationale Veröffentlichungsnummer: WO 2015/042628

(56) Entgegenhaltungen:
- WO-A1-2013/030024
- US-A- 2 418 000
- US-A1- 2002 049 214
- ZLATKOV A ET AL: "Synthesis, brain antihypoxic activity and cell neuroprotection of 1-substituted-3,7-dimethylxanthines", EUROPEAN JOURNAL OF MEDICINAL CHEMISTRY, EDITIONS SCIENTIFIQUE ELSEVIER, PARIS, FR, Bd. 35, Nr. 10, 1. Oktober 2000 (2000-10-01), Seiten 941-948, XP004220733, ISSN: 0223-5234, DOI: 10.1016/S0223-5234(00)01172-7
- DATABASE CA [Online] CHEMICAL ABSTRACTS SERVICE, COLUMBUS, OHIO, US; MORIKAWA, KOHEI ET AL: "Preparation of 3-methylolhydantoins by selective N-hydroxymethylation of (5-substituted)hydantoin", XP002733390, gefunden im STN Database accession no. 1989:212819 -& JP S63 275568 A (SHOWA DENKO K. K., JAPAN) 14. November 1988 (1988-11-14)

## Beschreibung

Die Erfindung betrifft Salze von Allantoin, Hydantoin und deren Derivate sowie deren Verwendung als Intumeszenzbeschichtung.

Brandschutzmittel auf Intumeszenzbasis wirken, in dem sie unter Hitzeeinwirkung einen Schutzschaum ausbilden, der das darunterliegende Substrat vor weiterem Wärmeintrag schützt und verhindert, dass weiteres Brennmaterial für das Feuer zur Verfügung steht. Ein Vorteil dieser Brandschutzmittel liegt darin, dass sie nicht in den Werkstoff eingearbeitet werden müssen, wodurch sie dessen Eigenschaften nicht verändern. Weiters sind sie einfach zu handhaben und können einfach vor Ort, auch nachträglich, aufgebracht werden.

Intumeszenzbeschichtungen beinhalten meistens drei Intumeszenz-relevante Komponenten: Eine Säurequelle, eine Kohlenstoffquelle und einen Gasbildner. Die Säurequelle führt zur Dehydration der Kohlenstoffquelle, die eine Kohleschicht bildet, welche durch den Gasbildner aufgeschäumt wird. Eine häufig verwendete Rezeptur für IntumeszenzBeschichtungen nutzt als Säurequelle Ammoniumpolyphosphat, als Kohlenstoffquelle Dipentaeryhtritol und als Gasbildner Melamin. Obwohl es bei den genannten drei Komponenten unzählige Variationsmöglichkeiten gibt, ist diese Rezeptur jene, die am besten untersucht ist und die besten Ergebnisse liefert.

Gerade auf dem Gebiet der Gasbildner wurde nach Alternativen gesucht und so wurden Hydantoine und Allantoine in ihrer Reinform in diversen Patentschriften (DE 28 44 132; A1; WO 2013/030024 A1) als mögliche Gasquelle für Intumeszenz-Lacke beschrieben. In anderen Veröffentlichungen werden Derivate der Hydantoine in eine Matrix einpolymerisiert. Das Dokument US 2,418,000 beschreibt 5,5-Dimethylhydantoin.

Direkte Abkömmlinge des Hydantoins sind die in Wasser nur schwer löslichen N,N'-Alkylbis(hydantoine), die antiseptische, germizide und fungizide Wirkung zeigen. Aus diesem Grund ist die effiziente Erzeugung dieser sehr kostengünstigen Substanzen auch Gegenstand neuerer Patentschriften. Doch wurde deren Einsatz als Brandschutzmittel/additiv noch nicht beschrieben. Aufgrund ihrer den Barbituraten analogen Acidität sind die Abkömmlinge des Hydantoins und Allantoins in wässrigen Alkalien leicht löslich. Die entsprechenden Salze dieser Verbindungen wurden allerdings nie eigens isoliert und genauer charakterisiert.

Im Rahmen der Erfindung wurde nun gefunden, dass Salze von Hydantoinen und Allantoinen sowie deren Derivate nicht nur unter Gasentwicklung, sondern auch ohne den zu erwartenden Zusatz einer Säure- und Kohlenstoffquelle in der Lage sind, eine eigenständige stabile durchgängige Schaumschicht zu bilden, weshalb sie sich als Single-Source Intumeszenz-Brandschutz eignen. Insbesondere die Alkalisalze, vorzugsweise Kalium- und Natriumsalze, haben sich als besonders geeignet erwiesen.

In einem ersten Aspekt betrifft die Erfindung eine Verbindung der Formel 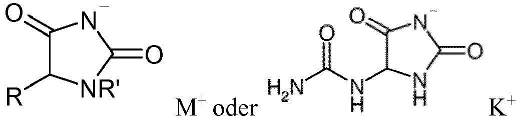 oder ein Hydrat davon,
wobei
R ausgewählt ist aus der Gruppe, bestehend aus:
   H, C₁-C₅-Alkyl und 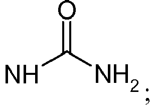
R' ausgewählt ist aus der Gruppe, bestehend aus: 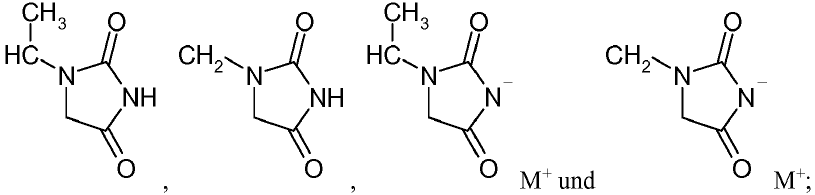 und
M⁺ entweder K⁺ oder Na⁺ ist.

Des Weiteren betrifft die Erfindung Verbindungen gemäß dem ersten Aspekt, aufweisend eine Formel, ausgewählt aus: 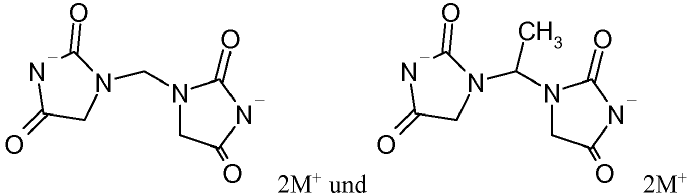 und den Hydraten davon.

In einem zweiten Aspekt betrifft die Erfindung ein Stoffgemisch, umfassend:
(i) eine erste Verbindung einer Formel, ausgewählt aus der Gruppe, bestehend aus: 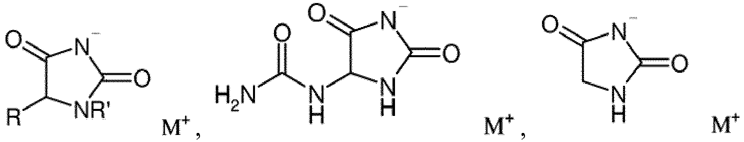 und den Hydraten davon, wobei R und R' wie im ersten Aspekt definiert sind und M⁺ = K⁺; und
(ii) eine zweite Verbindung einer Formel, ausgewählt aus der Gruppe, bestehend aus: 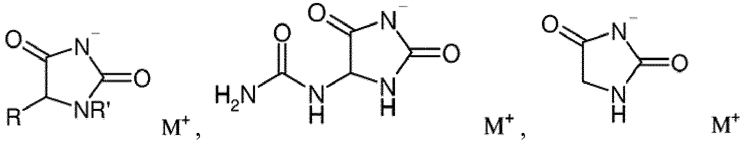 und den Hydraten davon, wobei R und R' wie im ersten Aspekt definiert sind und M⁺ = Na⁺.

Des Weiteren betrifft die Erfindung ein Stoffgemisch gemäß dem zweiten Aspekt, wobei die erste Verbindung Kaliumhydantoin und die zweite Verbindung Natriumhydantoin ist.

In einem dritten Aspekt betrifft die Erfindung eine Intumeszenzbeschichtung, umfassend eine Verbindung gemäß dem ersten Aspekt oder ein Stoffgemisch gemäß dem zweiten Aspekt. Bevorzugt besteht die Intumeszenzbeschichtung nur aus der Verbindung oder einer Mischung an Verbindungen der vorgenannten Art und gegebenenfalls Wasser.

In einem vierten Aspekt betrifft die Erfindung eine Intumeszenzbeschichtung, bestehend aus:
(a) einer Verbindung einer Formel, ausgewählt aus der Gruppe, bestehend aus: 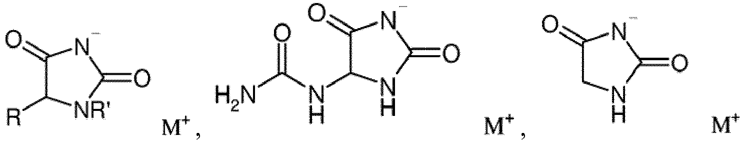 und den Hydraten davon, wobei R, R' und M⁺ wie im ersten Aspekt definiert sind, oder Gemischen der Verbindungen; und
(b) Wasser.

In einem fünften Aspekt betrifft die Erfindung ein Verfahren zum Aufbringen einer Intumeszenzbeschichtung auf ein Substrat, dadurch gekennzeichnet, dass eine wässrige Lösung einer Verbindung einer Formel, ausgewählt aus der Gruppe, bestehend aus: 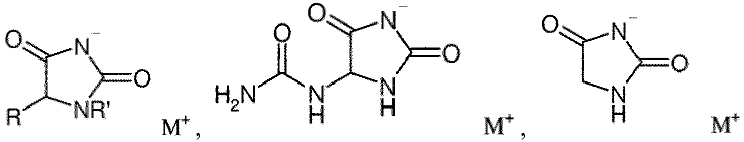 und den Hydraten davon, wobei R, R' und M⁺ wie im ersten Aspekt definiert sind, oder eines Stoffgemischs gemäß dem zweiten Aspekt auf das Substrat aufgebracht wird. Die Aufbringung kann z.B. durch Tränken, Aufspritzen, Aufdrucken usw. erfolgen.

In einem sechsten Aspekt betrifft die Erfindung die Verwendung einer Verbindung einer Formel, ausgewählt aus der Gruppe, bestehend aus: 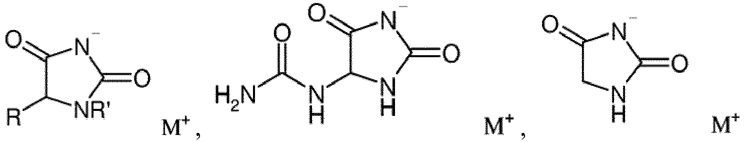 und den Hydraten davon, wobei R, R' und M⁺ wie im ersten Aspekt definiert sind, oder eines Stoffgemischs gemäß dem zweiten Aspekt als Intumeszenzbeschichtung.

Nachfolgend wird die brandschützende Wirksamkeit der vorgenannten Substanzen aufgezeigt (Beispiele, die nicht von den Ansprüchen umfasst sind, sind lediglich zu Vergleichszwecken angeführt):

**Tabelle 1: Ergebnisse zur Brandschutzwirkung.**

| **Konjugierte Säure des Anions** | **Kation** | **A:K** | **Wasser löslich** | **Brandtest** | **Generelle Eignung** |
|---|---|---|---|---|---|
| Allantoin | **Kalium** | **1:1** | + | **Starke Intumeszenz** | ++ |
| 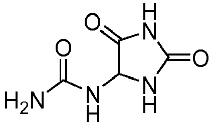 | **Natrium** | **1:1** | + | **Intumeszenz** | + |
| | **Calcium** | **2:1** | + | **Brennbar** | - |
| | **Al(OH)₂*** | **1:1** | - | - | - |
| Hydantoin | **Kalium** | **1:1** | + | **Starke Intumeszenz** | ++ |
| 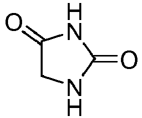 | **Natrium** | **1:1** | + | **Intumeszenz** | + |
| | **Calcium** | **2:1** | - | - | - |
| | Tetramethylguanidinium | **1:1** | + | **Brennbar** | - |
| | 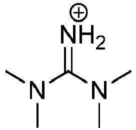 | | | | |
| | Tetramethylammonium | **1:1** | + | **Brennbar** | - |
| | 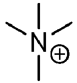 | | | | |
| | Tetrakis(hydroxymethylphosphonium) | **1:1** | + | **Brennbar** | ∼ |
| | 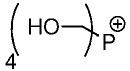 | | | | |
| | ***mögliche Formaldehydquelle*** | | | | |
| 1,1'-(Methylen) bishydantoin | **Kalium** | **1:2** | + | **Starke Intumeszenz** | + |
| 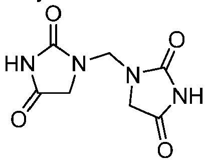 | **Natrium** | **1:2** | + | **Intumeszenz** | + |
| | **Calcium** | **1:1** | ∼ | **Brennbar** | - |
| | Tetrakis(hydroxymethyl-phosphonium) | **1:2** | + | **Selbstlöschend; Starke Rauchbildung** | ∼ |
| ***mögliche Formaldehydquelle*** | 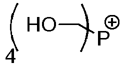 | | | | |
| | ***mögliche Formaldehydquelle*** | | | | |
| 1,1'-(Methylmethylen) bishydantoin | **Kalium** | **1:2** | + | **Starke Intumeszenz** | + |
| 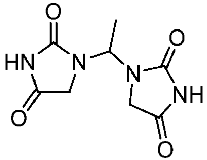 | **Natrium** | **1:2** | + | **Starke Intumeszenz** | + |
| | **Calcium** | **1:1** | + | **Brennbar** | - |

| | | | | | |
|---|---|---|---|---|---|
| *Dihydroxyalumniumallantoinat (ALDA CAS:5579-81-7) DE3034506A1; ++: sehr gute Eigenschaften; +: gute ; ∼: neutrale; -: schlechte; A:K gibt das Verhältnis von Anion zu Kation an | | | | | |

- Fig. 1: zeigt die Stoffmenge im Zellstoff nach 5 Min. Tränkung bei verschiedenen Lösungskonzentrationen.
- Fig. 2: zeigt die Masse im Zellstoff nach 5 Min. Tränkung bei verschiedenen Lösungskonzentrationen.
- Fig. 3: zeigt eine Bildserie des Abbrands von Kalium(I)hydantoinat, Natrium(I)hydantoinat, Dikalium(I)N,N'-Methylenbishydantoinat, Dinatrium(I)N,N'-Methylenbishydantoinat.
- Fig. 4: zeigt Kalium(I)hydantoin (5) bei verschiedenen Konzentrationen im Zellstoff.
- Fig. 5: zeigt guten konsistenten Schaum (links) und zu starkes Schäumen (rechts).

### Synthese

### 1) Allgemeines zur Synthese

Die Synthese der Salze (siehe auch Tabelle 1) beruht auf einer Säure-Base Reaktion aus der konjugierten Säure mit dem entsprechenden Hydroxid des gewünschten Kations in wässrigem Medium. Alle Verbindungen werden in einer ausgezeichneten Reinheit meistens als Hydrate erhalten. Nur die Kombination mit Tetrakis(hydroxymethyl)phosphonium lieferte ein Material, das lediglich eine Mischung aus Edukt und Produkt darstellte. Zusätzlich besitzt es einen leichten unangenehmen Eigengeruch, was ein Ausschlusskriterium für manche Anwendungen ist. Außerdem konnte die vielversprechende Methylenbisbarbitursäure nicht in geeignete Salze überführt werden und dient deshalb als Gegenbeispiel.

Durch die Verwendung von Wasser als Lösungsmittel und den Einsatz nicht schädlicher Edukte kann die Synthese als sehr umweltfreundlich angesehen werden. Die einzige Ausnahme stellt die Synthese des N,N'-Methylenbishydantoins dar, da bei dessen Herstellung Formaldehyd verwendet wird. Obwohl die hergestellte Verbindung kein Formaldehyd abspalten sollte, führt die Verwendung von Formaldehyd in der Synthese zu einer negativen Bewertung in puncto Umweltfreundlichkeit.

### 2) Stöchiometrie

Bei Hydantoin und Allantoin handelt es sich um einprotonige Säuren und die Methylenverbrückten Bishydantoine sind zweiprotonige Säuren. Dementsprechend wurden die stöchiometrischen Einsatzmengen ausgewählt. Im Falle des Dinatrium(I)1,1'-Methylenbishydantoins kristallisierte aus der Hauptmenge, die in einer 1:2 Säure:Base Stöchiometrie vorlag, ein Salz mit einer 1:1 Stöchiometrie aus. Aus diesem Grund wurde versucht, diese Mononatrium Modifikation gezielt herzustellen, was erfolglos war (IR und XPRD Analysen). Logischerweise wurde auch versucht, aus dem Dikalium(I)1,1'-Methylenbishydantoin eine Monomodifikation zu gewinnen, doch konnte auch diese nicht hergestellt werden (IR und XPRD Daten).

### Löslichkeit

Organische Lösungsmittel tragen zur Umweltverschmutzung bei und deshalb ist es wünschenswert, Wasser als Basis für Reaktionen oder auch Lacke zu verwenden. Die Reinsubstanzen von Hydantoin, Allantoin und die Kondensationsprodukte mit Aldehyden sind schlecht wasserlöslich, aber die Überführung in ihre Salze mit geeigneten Kationen kann sie wasserlöslich machen. Wir beobachteten, dass vor allem die Kombination mit Alkalimetallen zu einer sehr guten Löslichkeit führte.

Im Falle der Salzbildungsgleichgewichte mit bestimmten Stickstoffbasen (Meglumin oder Ammoniak) konnte gezeigt werden, dass die Derivate des Hydantoins und Allantoins während der Auftrocknung sich nicht als Salz, sondern als neutrale Ausgangsverbindungen abscheiden. Damit können die neutralen Ausgangsverbindungen temporär wasserlöslich werden, wodurch sie für andere Auftragsmethoden zugänglich werden.

### Abbrandversuche

### 1) Auswahl der Zielsubstanzen

Ein wichtiges Kriterium für die Auswahl der Substanzen für eingehendere Studien, war deren Wasserlöslichkeit. Ausgeschlossen wurden vor allem Verbindungen, die höhere Kationen als die Alkalimetalle enthalten, da diese nur sehr schlecht löslich sind. Trotzdem wurde zumindest für zwei wasserunlösliche Salze versucht, sie als Suspensionen aufzubringen. Ausgewählt wurden dafür ALDA und Calcium(II)hydantoinat. Doch konnte von ALDA keine ausreichende Menge (20 mg) aufgebracht werden und das Calcium(II)hydantoinat ergab keine glatte Oberfläche und versagte beim Brandtest.

### 2) Testmedium

Die Substanzen schäumen zwar in Reinform auf, aber es musste getestet werden, ob sie diese Eigenschaft auch nach Auf-/Einbringung in ein Substrat besitzen. Dies traf bei Furnieren und auch Zellstoffen zu. Der Zellstoff wurde ausgewählt, da dieser definierte Parameter besitzt und leicht erhältlich war. Außerdem führten die Proben bei Furnieren zu einer leichten Vergilbung, weshalb diese als Testmedium ungeeignet schienen.

### 2.1) Allgemeine Aufbringung der Zielsubstanzen

Für die Prüfung der brandhemmenden Wirkung wurden Blättchen aus dem Zellstoff in der Größe 3x3 cm ausgeschnitten und anschließend in eine 20%ige Lösung der Zielsubstanz für 300 s eingelegt. Diese Konzentration wurde gewählt, da Vorstudien gezeigt haben, dass bei dieser Konzentration die Ergebnisse sich am besten vergleichen lassen. Des Weiteren wurde darauf geachtet, dass die Blättchen über die angegebene Zeit vollständig benetzt bleiben. Anschließend wurden die noch feuchten Proben in einem Exsikkator bis zur Gewichtskonstanz getrocknet.

### 2.2) Konzentrationsabhängige Aufbringung der Kalium- und Natriumsalze des Hydantoins und N,N'-Methylenbishydantoins

Es wurden von den Hydantoin- und 1,1'-Methylenbis(hydantoin)salzen Lösungen in H₂O_{dest} mit verschiedenen Konzentrationen erstellt. Die Konzentrationen wurden in 5% - Schritten von 5% bis maximal 40% - falls die Löslichkeit des Salzes dies zuließ - ansonsten bis zur maximal erreichbaren Konzentration, gewählt (alle Angaben in Masseprozent).

In je 20 ml der Lösung in einer Kristallisationsschale wurden anschließend 5 cm x 5 cm große Blättchen des Zellstoffes (2,055 g +/- 0,007 g) für exakt 300 s so in die Lösung eingelegt, dass sich deren Oberseite vollständig unter dem Flüssigkeitsspiegel befand. Die Lösung befand sich dabei bereits in der Kristallisationsschale, als der Zellstoff zugegeben wurde, um auch die Unterseite gut zu benetzen. Anschließend wurden die Blättchen im Vakuum bis zur Gewichtskonstanz getrocknet.

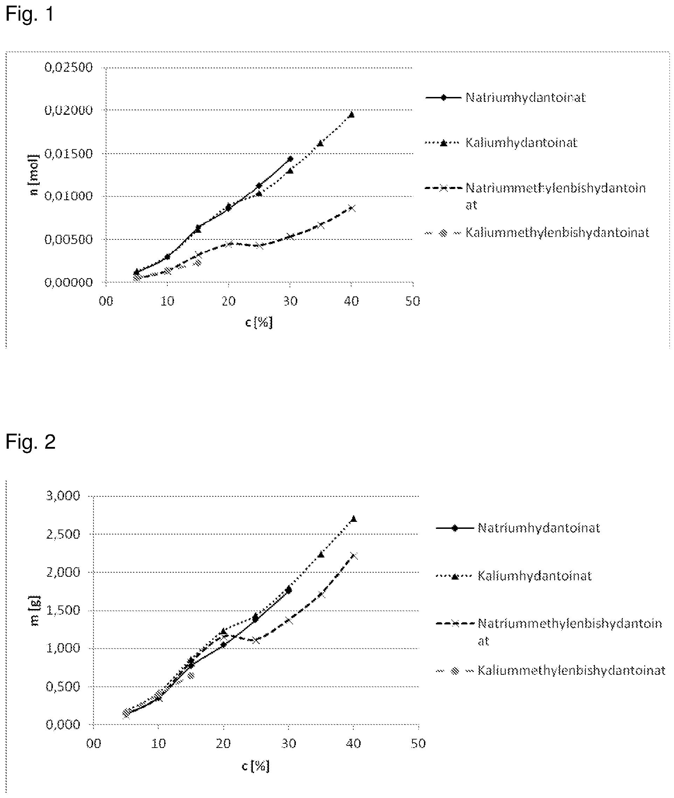

Die nachfolgenden Fig. zeigen die Stoffmenge (Fig. 1) bzw. die Masse (Fig. 2) an Probe, welche nach 300 s Tränkung im Zellstoff verblieben. Während der Zellstoff in der Lösung lag, quoll er stark auf. Dieser Effekt ging beim Trocknen wieder zurück, allerdings fiel deutlich auf, dass mit steigender Konzentration auch die Dicke des getrockneten Blättchens stieg. Außerdem wurden die Blättchen mit steigender Konzentration der Lösung nach dem Trocknen härter.

### 3) Intumeszenzeigenschaften im Zellstoff:

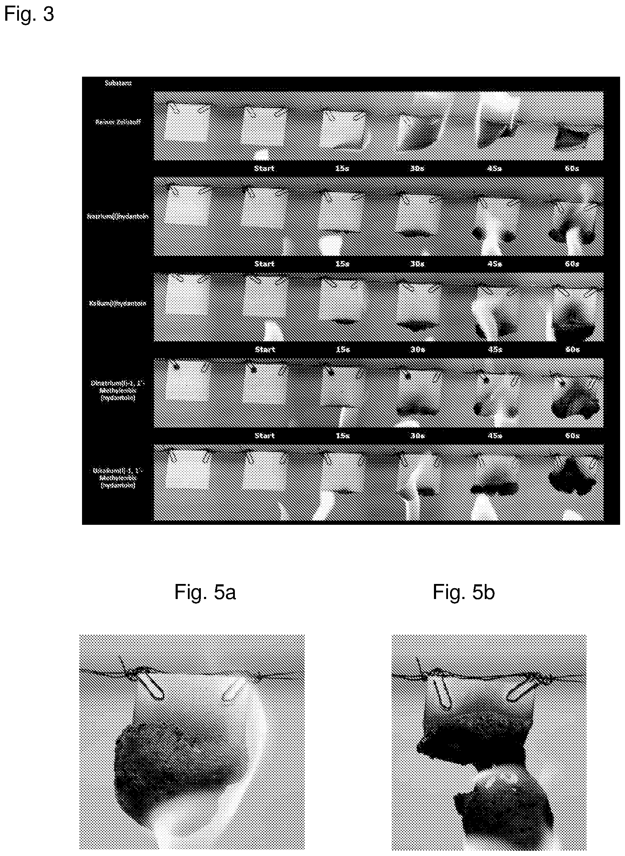

Die wie vorher beschriebenen Blättchen wurden in einer Selbstkonstruktion eingespannt und es wurde mittels eines Bunsenbrenners versucht, diese zu entzünden. Wenn die Probe sich entzündete, wurde der Bunsenbrenner entfernt. Im Falle einer Auslöschung der Flamme wurde die Probe wieder entzündet. Proben, die sich nicht entzündeten, wurden eine gewisse Zeit beflammt. Der Versuch wurde gefilmt und diente als Grundlage für die Bewertung der Brandschutzwirkung der Proben (Beispiele Fig. 3).

### 3.1) Einfluss der Anionen

Wir stellten die eigenständige Intumeszenz zum ersten Mal bei dem Dinatrium(I)N,N'-Methylenbishydantoin fest, als wir dessen thermisches Verhalten in einem Mikroskop mit Heizplatte untersuchten. Um die einzelnen Strukturelemente zu identifizieren, die die eigenständige Intumeszenz beeinflussen, reduzierten wird die Struktur des Anions auf das Hydantoin und stellten fest, dass diese eine stärkere Schaumbildung zeigten als die N,N'-Methylenbishydantoine. Erweiterung der Struktur des Hydantoins auf das Allantoin brachte ähnliche Ergebnisse. Außerdem untersuchten wir den Einfluss eines Substituenten an der Methylenbrücke des N,N'-Methylenbishydantoins und fanden, dass die Veränderung der Methylenbrücke die Intumeszenz-Wirkung weder verbessert noch beeinträchtigt. Dadurch konnte gezeigt werden, dass eine Modifikation an der Methylenbrücke eine Möglichkeit bietet, zusätzliche Funktionalitäten einzuführen, ohne dass der Intumeszenzeffekt verloren geht.

### 3.2) Einfluss der Kationen

Alle Experimente zeigten, dass die Wahl des Kations einen großen Einfluss auf die Intumeszenzwirkung der Verbindungen hat.

Ein Versuchsexperiment mit einer Intumeszenzbeschichtung aus einer 1:1-Mischungsphase aus Kalium- und Natriumhydantoinat ergab, dass die unterschiedlichen Kationen unterschiedliche Intumeszenztemperaturprofile aufweisen, was die Wirkung der Intumeszenzbeschichtung ausweitet.

Um die eigenständige Intumeszenz im Zellstoff auszubilden, ist ein anorganisches Kation sinnvoll. Die Kombinationen der oben angeführten Anionen mit Kalium und Natrium bildeten immer einen Schutzschaum aus, der sich aber in Volumen und Stabilität abhängig vom Anion unterschied.

Werden anorganische Kationen mit einer größeren Ladung verwendet, wie zum Beispiel Calcium, wird eine ineffiziente oder keine Schutzschaumbildung beobachtet.

Die Verwendung der stickstoffhaltigen organischen Kationen 1,1,3,3,-Tetramethylguandinium und Tetramethylammonium zielte darauf ab, bei der Verbrennung mehr Gase freizusetzen, um mehr Schaumvolumen zu produzieren. Doch zeigte sich dieser Effekt nicht und die Proben verbrannten ähnlich wie der reine Zellstoff. Trotzdem könnten sich die Substanzen als wasserlösliche Gasbildner für 3-Komponenten IntumeszenzBeschichtungen eignen.

Aufgrund der Tatsache, dass sich Phosphor positiv auf die brandhemmende Wirkung von Schutzanstrichen auswirkt, wurde versucht diesen als Tetrakis(hydroxymethyl)phosphonium zu inkorporieren. Die Kombinationen mit diesem Kation waren selbstlöschend, konnten aber immer wieder für kurze Zeit entzündet werden. Zusätzlich bildete sich dabei sehr viel unangenehm riechender Rauch.

### 3.3) Einfluss der Konzentration:

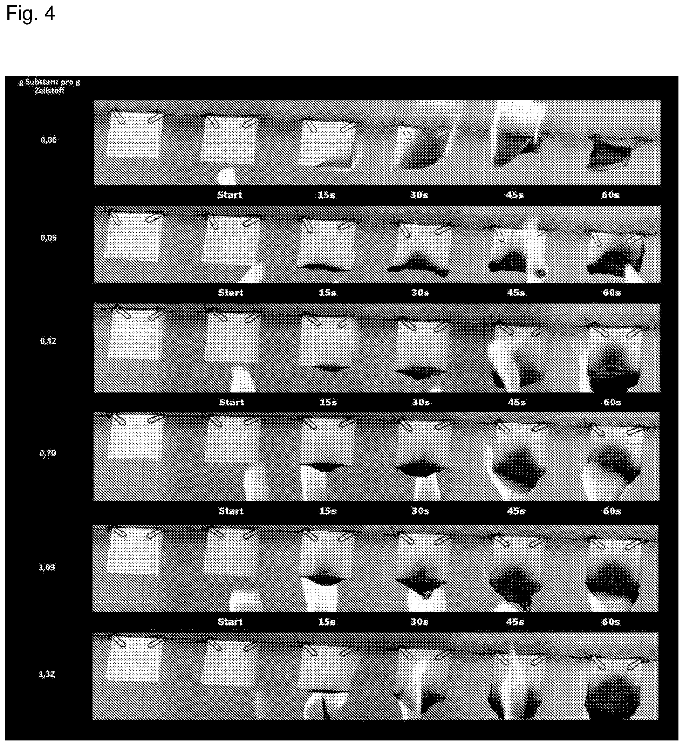

Für die Substanzen, die eine eigenständige Intumeszenz zeigten, steigt das Volumen des Schaums mit zunehmender Konzentration an (Beispiel Kalium(I)hydantoinat Fig. 4). Im Falle des Kalium(I)hydantoinats zeigte sich, dass höhere Konzentrationen auch einen negativen Einfluss haben können, da die Schaumbildung bei höheren Lösungskonzentrationen ab 30% so stark wird, dass der Schaum durchreißt und abfällt, wodurch ein großer Teil der Schutzwirkung verloren geht (Fig. 5).

Die N,N'-(Alkyl)bishydantoine zeigen bei niedrigerer Konzentration weniger Schaumbildung als reines Hydantoin, doch wird die Schaumbildung mit steigender Konzentration nie so stark, dass sie zum Durchreißen des Schaumes führt. Das gilt nur im Falle des verwendeten Zellstoffes. Für andere zu schützende Substanzen muss es von Fall zu Fall getestet werden.

### Experimenteller Teil

### N,N'-Methylenbishydantoin (1); (US 5,554,762)

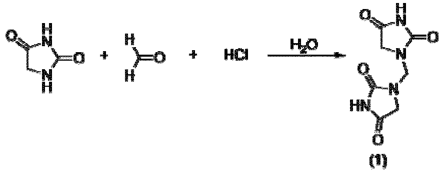

In einem 500 ml Kolben wurden Formaldehyd wässr. Lsg. 37% (53.1 mL, 0.510 mol), Hydantoin (101.6 g, 1.020 Mol), HCl konz. 37% (150 mL) und H₂O_{dest}. (100 mL) vereinigt und 72 h gerührt. Anschließend wurde der weiße Feststoff abgenutscht und mit Wasser gewaschen. Nachfolgende Trocknung bei 120°C für einen Tag ergab N,N'-Methylenbishydantoin (1) (96.2 g, 89%). mp 300°C. δ_{H} (*DMSO-d₆*, 300 MHz) 3.93 (s, 4H, C6H₂, C6'H₂), 4.74 (s, 2H, C1H₂), 10.84 (s, 2H, N3H, N3'H). δ_{C} (*DMSO-d₆*, 75 MHz): 48.5 (C1), 50.1 (C6,C6'), 157.4 (C4,C4'), 171,8 (C3, C3'). νₘₐₓ(neat)/cm⁻¹ 3196 (m), 3074 (w), 1752 (m), 1609 (s), 1474 (m), 1454 (s), 1424 (m), 1376 (s), 1333 (m), 1263 (m), 1210 (s), 1171 (m), 1150 (s), 1098 (m), 984(w), 961 (m), 896 (m), 843 (w), 760 (m), 714 (s), 696 (s), 639 (s), 599 (s), 572 (s), 444 (m).

### Natrium(I)N,N'-methylenbishydantoinat (2)

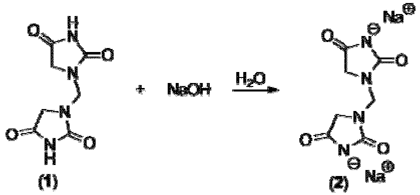

In einem 1 L Kolben wurden N,N'-Methylenbishydantoin (95.6 g, 0.450 mol) (1) in H₂O_{dest}. (250 mL) suspendiert und Natriumhydroxid (36.1 g, 0.900 mol) wurde portionsweise zugegeben. Nachdem der Großteil gelöst war, wurde die Lösung filtriert und das Lösungsmittel am Rotationsverdampfer entfernt. Der Rückstand wurde 8 h bei 50°C am Hochvakuum getrocknet und verfestigte sich nach einigen Tagen zu dem Dinatrium-N,N'-methylenbishydantoinat·Tetrahydrat (124.0 g). Langsame Kristallisation aus Methanol/H₂O_{dest}. führt zu Mononatrium-N,N'-methylenbishydydantoinat·Sesterhydrat (·2.5 H₂O). δ_{H} (*D₂O*, 300 MHz) 3.93 (s, 2H, C6H₂, C6'H₂), 4.90 (s, 4H, C1H₂). δ_{C} (*D₂O*, 75 MHz): 46.4 (C1), 49.2 (C6,C6'), 170.6 (C5,C5'), 187.0 (C3, C3'). νₘₐₓ(neat)/cm⁻¹ 3355 (bm), 3010 (bm), 2737 (w), 1763 (w), 1685 (m), 1569 (s), 1460 (m), 1449 (m), 1406 (m), 1347 (s),1264 (m), 1202 (s), 1154 (s), 1113 (m), 994 (w), 964 (m), 909 (w), 855 (w), 786 (s), 760 (m), 686 (m), 617 (m), 582 (m).

### Kalium(I)N,N'-methylenbishydantoinat (3)

N,N'-Methylenbishydantoin **(1)** (80.0 g, 0.380 mol) und Kaliumhydroxid (42.3 g, 0.750 mol) in H₂O_{dest}. (250 mL) ergaben weiß kristallines Kalium(I)N,N'-methylenbishydantoinat·Tetrahydrat (**3**) (126.0 g). Langsame Kristallisation aus H₂O_{dest}. führte zum hydratisierten Dikaliumsalz. δ_{H} (*D₂O*, 300 MHz) 3.95 (s, 2H, C6H₂, C6'H₂), 4.91 (s, 4H, C1H₂). δ_{C} (*D₂O*, 75 MHz): 43.8 (C1), 46.4 (C6,C6'), 166.1 (C5,C5'), 182.4 (C3, C3'). νₘₐₓ(neat)/cm⁻¹: 3443 (m), 3256 (bm), 3122 (bm), 1796 (m), 1674 (m), 1598 (s), 1570 (s), 1469 (m), 1444 (s), 1423 (w), 1399 (m), 1368 (s), 1331 (s), 1267 (m), 1202 (s), 1160 (s), 996 (w), 960 (m), 896 (w), 865 (m), 792 (s), 765 (m), 700 (s), 681 (m), 616 (s), 571 (s), 494 (m), 432 (s).

### Calcium(II)N,N'-methylenbishydantoinat (4)

N,N'-Methylenbishydantoin **(1)** (1.0 g, 0.005 mol) und Calciumhydroxid (0.4 g, 0.005 mol) in H₂O_{dest}. (5 mL) ergaben Calcium(II)N,N'-methylenbishydantoinat **(4)** (0.95 g, 81%) als weiße, kristalline Substanz. δ_{H} (*D₂O*, 300 MHz): 3.93 (s, 2H, C6H₂, C6'H₂), 4.90 (s, 4H, C1H₂). δ_{C} (*D₂O*, 75 MHz): 43.8 (C1), 46.6 (C6,C6'), 167.5 (C5,C5'), 184.0 (C3, C3'). νₘₐₓ(neat)/cm⁻¹: 3512 (bm), 3373 (bm), 3159 (bm), 2927 (bm), 1682 (m), 1567 (s), 1454 (s), 1423 (m), 1405 (s), 1363 (s), 1335 (s), 1274 (m), 1232 (m), 1215 (s), 1194 (s), 989 (w), 957 (w), 925 (w), 869 (m), 788 (s), 709 (m), 661 (m), 622 (s), 580 (s), 418 (m).

### Barium(II)N,N'-methylenbishydantoinat (5)

N,N'-Methylenbishydantoin **(1)** (1.0 g, 0.005 mol) und Bariumhydroxid·Octahydrat (1.5 g, 0.005 mol) in H₂O_{dest}. (10 mL) unter Ar(g) ergaben Barium(II)N,N'-methylenbishydantoinat **(5)** (1.4 g, 86%) als klares Öl. δ_{H} (*D₂O*, 300 MHz): 3.92 (s, 4H, C6H₂, C6'H₂), 4.89 (s, 2H, C1H₂). δ_{C} (*D₂O*, 75 MHz) 43.8 (C1), 46.7 (C6,C6'), 168.0 (C5,C5'), 184.4 (C3, C3'). νₘₐₓ (neat)/cm⁻¹: 3337 (b), 3246 (b), 2931 (b), 1672 (w), 1547 (s), 1442 (m), 1401 (m), 1342 (m), 1307 (m), 1206 (s), 965 (w), 860 (w), 784 (m), 667 (w), 610 (m), 419 (w).

### Bis(1,1,3,3-Tetramethylguandinium)N,N'-methylenbishydantoinat (6)

N,N'-Methylenbishydantoin **(1)** (1.0 g, 0.005 mol) und 1,1,3,3-Tetramethylguanidin (1.2 mL, 0.010 mol) in H₂O_{dest}. (5 mL) ergaben Bis(1,1,3,3-tetramethylguanidinium)N,N'-methylenbishydantoinat **(6)** (2.0 g, 96%) als wachsartige, gelbliche Substanz. δ_{H} (*d₆-DMSO*, 300 MHz) 2.78 (s, 24H, 8x TMG-CH₃), 3.54 (s, 4H, C6H₂, C6'H₂), 4.64 (s, 2H, C1H₂), 5.09 (bs, TMG-NH₂). δ_{C} (*d₆-DMSO*, 75 MHz) 39.3 (8x TMG-CH₃), 49.2 (C1), 51.1 (C6,C6'), 163.2 (TMG-C1), 166.7 (C5,C5'), 179.7 (C3, C3'). νₘₐₓ(neat)/cm⁻¹ 3511 (w), 2935 (bm), 1702 (w), 1557 (s), 1464 (m), 1403 (m), 1328 (s), 1279 (m), 1192 (s), 1176 (s), 1090 (m), 1065 (m), 1036 (m), 952 (w), 880 (w), 790 (m), 711 (m), 688 (m), 653 (m), 613 (s), 580 (m), 540 (m), 483 (w), 410 (w).

### Bis(tetramethylammonium)N,N'-methylenbishydantoinat (7)

N,N'-Methylenbishydantoin **(1)** (1.0 g, 0.005 mol) und Tetramethylammoniumhydroxid·Pentahydrat (1.7 g, 0.010 mol) in H₂O_{dest}. (5 mL) ergaben Bis(tetramethylammonium)N,N'-methylenbishydantoinat (7) (1.6 g, 95%) als wachsartige, kristalline Substanz. mp 45°C. NMR: δ_{H} (*D₂O*, 300 MHz): 3.21 (s, 24H, 8x N-CH₃), 3.91 (s, 4H, C6H₂, C6'H₂), 4.89 (s, 2H, C1H₂). δ_{C} (*D₂O*, 75 MHz): 43.8 (C1), 46.61 (C6,C6'), 50.0 (N-CH₃), 168.04 (C5,C5'), 184.33 (C3, C3'). νₘₐₓ(neat)/cm⁻¹ 3441 (m), 3284 (bm), 3025 (w), 1683 (m), 1569 (s), 1488 (m), 1448 (m), 1396 (w), 1301 (m), 1259 (m), 1193 (s), 994 (w), 949 (s), 851(w), 794 (m), 690 (m), 688 (m), 611 (s), 578 (s), 456 (w), 414 (w).

### Bis(N-methyl-D-glucamin)N,N'-methylenbishydantoinat (8)

N,N'-Methylenbishydantoin **(1)** (1.0 g 0.005 mol) und N-Methyl-D-Glucamin (1.8 g, 0.010 mol) in H₂O_{dest}. (10 mL) ergaben Bis(N-methyl-D-glucamin)N,N'-methylenbishydantoinat **(8)** (2.8 g, 98%) als wachsartige, weiße Substanz. δ_{H} (*d₆-DMSO*, 300 MHz): 2.27 (s, 6H, N-CH₃), 2.56 *(d,* 4 H, J= 4.4 Hz, N-CH₂), 3.37 (*m*, CH-OH), 3.47 (*m*, CH-OH), 3.63 (m, CH-OH), 3.83 (b, N-H), 3.91 (C6H₂, C6'H₂), 4.73 (s, 2H, C1H₂). δ_{C} (*d₆-DMSO*, 75 MHz) 35.9 (N-CH₃), 48.6 (C1), 50.1 (C6,C6'), 52.7 (N-CH₂), 63.8 (CH₂-OH), 70.5 (d, CH-OH), 70.9 (CH-OH), 71.2 (CH-OH), 157.9 (C5,C5'), 172.2 (C3, C3'). νₘₐₓ(neat)/cm⁻¹ 3314 (s), 3233 (s), 2979 (m), 2951 (m), 2918 (m), 2730 (m), 2525 (w), 1764 (w), 1715 (m), 1660 (m), 1587 (s), 1475 (m), 1437 (s), 1419 (s), 1376 (m), 1304 (m), 1210 (m), 1174 (m), 1152 (m), 1118 (m), 1096 (s), 1074 (s), 1048 (s), 1016 (s), 971 (w), 943 (w), 907 (m), 885 (m), 864 (m), 845 (m), 764 (w), 687 (m), 645 (s), 599 (s), 573 (m), 516 (m), 463 (m).

### Lithium(I)N,N'-methylenbishydantoinat (9)

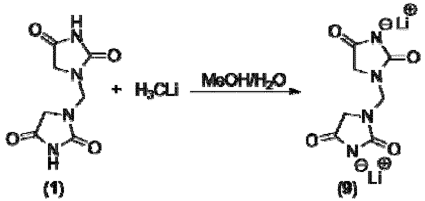

In einem 50 mL Kolben wurden Lithiummethanolat (0.3 mL, 0.008 mol) in Methanol (10 mL) gelöst und N,N'-Methylenbishydantoinat **(1)** (0.7 g, 0.004 mol) wurde zugegeben. Der weiße Feststoff wird zuerst angelöst und beginnt dann wieder auszufallen. Zugabe von H₂O_{dest}. (10 mL) löste den Feststoff vollständig. Das Lösungsmittel wurde am Rotationsverdampfer entfernt und am Hochvakuum für 3 h getrocknet, was Lithium(I)N,N'-methylenbishydantoinat **(9)** (0.7 g, 91%) als weißes Pulver ergab. νₘₐₓ(neat)/cm⁻¹ 3447 (bw), 3346 (bm), 1684 (m), 1558 (s), 1453 (m), 1430 (m), 1405 (m), 1376 (m), 1344 (m), 1283 (m), 1209 (s), 1179 (m), 1115 (w), 999 (w), 966 (w), 935 (m), 872 (w), 788 (m), 716 (m), 614 (s), 579 (m), 423 (w).

### Silber(I)N,N'-methylenbishydantoinat (10)

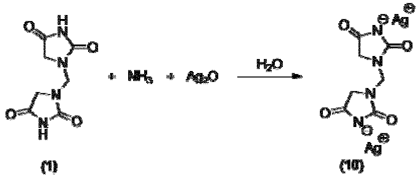

In einem 50 mL Kolben wurden N,N'-Methylenbishydantoin **(1)** (1.0 g, 0.005 mol) in H₂O_{dest}. (10 mL) suspendiert und mit Ammoniak Lsg. 25% (1.4 ml, 0.019 mol) versetzt, woraufhin sich eine klare Lösung bildet. Der Kolben wurde vor Licht geschützt und Silberoxid (1.1 g, 0.005 mol) wurde dazugegeben. Die Reaktionslösung wurde über Nacht gerührt und der metallisch graue Niederschlag abgenutscht und mit H₂O_{dest}. gewaschen. Anschließende Trocknung für 3 h am Hochvakuum ergab Silber(I)N,N'-methylenbishydantoinat **(10)** (1.8 g, 91%) als graues Pulver. νₘₐₓ(neat)/cm⁻¹ 3334 (w), 3298 (m), 3245 (w), 3162 (m), 1692 (m), 1630 (s), 1606 (s), 1461 (s), 1441 (m), 1421 (m), 1351 (s), 1322 (s), 1275 (m), 1257 (m), 1213 (s), 1183 (s), 1165 (m), 964 (m), 937 (w), 884 (w), 787 (m), 705 (m), 648 (m), 619 (s), 604 (s), 585 (m), 468 (w), 421 (m).

### Bis[tetrakis(hydroxymethyl)phosphonium]N,N'-methylenbishydantoinat (11)

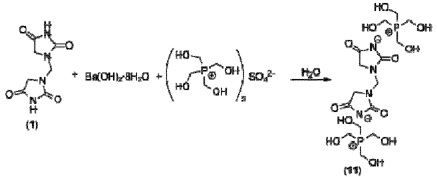

In einem 50 mL Kolben wurden N,N'-Methylenbishydantoin **(1)** (1.0 g, 0.005 mol) in H₂O_{dest}. (10 mL) suspendiert und mit Bariumhydroxid·Octahydrat (1.5 g, 0.005 mol) versetzt, woraufhin sich fast alles löste. Anschließend wurde Bis[tetrakis(hydroxymethyl)phosphonium]sulfat Lsg. 72.5% (1.9 mL, 0.005 mol) zugegeben, dabei bildet sich ein weißer Niederschlag. Die Suspension wurde über Nacht gerührt und der weiße Niederschlag abgenutscht. Der Niederschlag wurde verworfen und die Lösung am Rotationsverdampfer eingeengt. Trocknung am Hochvakuum für 3 h ergab Bis[tetrakis(hydroxymethyl)phosphonium]N,N'-methylenbishydantoinat **(11)** (1.7 g, 70%) in einer Mischung mit nicht umgesetztem Edukt als wachsartige Substanz. νₘₐₓ(neat)/cm⁻¹ 3197 (m), 2970 (bm), 2823 (bm), 1763 (m), 1689 (m), 1595 (m), 1451 (s), 1372 (s), 1264 (m), 1210 (s), 1169 (m), 1140 (s), 1038 (s), 959 (m), 895 (m), 761 (m), 693 (s), 638 (m), 598 (m), 571(s), 444 (m).

### N,N'-(Methylmethylen)bishydantoin (12)

Hydantoin (100.1 g, 1.0 mol) wurde in H₂O_{dest}. (100 mL) suspendiert, mit Acetaldehyd (42.4 mL, 0.8 mol) versetzt und langsam wurde HCl (150 mL) zugegeben. Die Suspension wurde für 24 h gerührt, wobei sie eine braune Färbung annahm; bei der anschließenden Filtration konnte die braune Farbe vollständig mit H₂O_{dest}. entfernt werden. Trocknung am HV für 6 h ergab N,N'-(Methylmethylen)bishydantoin **(12)** (82.2 g, 73%). Kristalle für Röntgenstrukturanalysen konnten durch langsame Kristallisation aus H₂O_{dest.}/NH₃ gewonnen werden. δ_{H} (DMSO-d₆, 300 MHz) 1.45 *(d,* 3H, J= 6.9 Hz, -CH₃), 4.00 *(d,* 4H, J= 3.0 Hz, C6H₂, C6'H₂), 5.68 (*q,* 1H, J= 6.9 Hz, C1H), 10.80 (s, 2H, N3H, N3'H). δ_{C} (DMSO-d₆, 75 MHz) 16.5 (-CH3), 48.3 (C6, C6'), 55.8 (C1), 156.2 (C5, C5'), 171.7 (C3, C3'). νₘₐₓ (neat)/cm⁻¹ 3137 (w), 3040 (w), 2956 (w), 2771 (w), 1758 (m), 1689 (s), 1476 (m), 1448 (m), 1415 (m), 1389 (m), 1348 (w), 1323 (m), 1251 (m), 1232 (m), 1213 (m), 1146 (m), 1118 (m), 1068 (m), 1053 (m), 986 (w), 907 (w), 894 (w), 842 (w), 809 (w), 758 (m), 712 (w), 687 (m), 658 (m), 616 (m), 594 (m), 576 (w), 471 (m), 431 (s).

### Natrium(I)N,N'-(methylmethylen)bishydantoinat (13)

N,N'-(Methylmethylen)bishydantoin **(12)** (20.0 g, 0.09 mol) wurde in H₂O_{dest}. (50 mL) suspendiert und mit Natriumhydroxid (7.1 g, 0.16 mol) versetzt. Nachdem alles gelöst war, wurde das Lösungsmittel am Rotationsverdampfer entfernt und eine nachfolgende Trocknung am Hochvakuum bei 50°C ergab festes, weißes Natrium(I)N,N'-(methylmethylen)bishydantoinat **(13)** (23.2 g, 97%). δ_{H} (D₂O, 300 MHz) 1.46 (*d*, 3H, J= 7 Hz, -CH₃), 3.82 (*m*, 4H, C6H₂, C6'H₂), 5.85 *(m,* 1H, C1H). δ_{C} (D₂O, 75 MHz) 19.6 (-CH₃), 52.4 (C6, C6'), 58.7 (C1), 174.9 (C5, C5'), 192.4 (C3, C3'). νₘₐₓ(neat)/cm⁻¹ 3257 (m), 2162 (w), 1682 (m), 1565 (s), 1441 (m), 1373 (s), 1325 (s), 1202 (s), 1072 (w), 1053 (w), 986 (m), 829 (m), 789 (m), 748 (m), 661 (m), 611 (m), 432 (m).

### Kalium(I)N,N'-(methylmethylen)bishydantoinat (14)

N,N'-(Methylmethylen)bishydantoin **(12)** (20.0 g, 0.09 mol) wurde in H₂O_{dest}. (50 mL) suspendiert und mit Kaliumhydroxid (9.9 g, 0.16 mol) versetzt. Nachdem alles gelöst war, wurde das Lösungsmittel am Rotationsverdampfer entfernt und eine nachfolgende Trocknung am Hochvakuum bei 50°C ergab festes, weißes Kalium(I)N,N'-(methylmethylen)bishydantoinat **(14)** (21.2 g, 79%). δ_{H} (D₂O, 300 MHz) 1.46 (*d*, 3H, J= 7 Hz, -CH₃), 3.82 (*m*, 4H, C6H₂, C6'H₂), 5.85 *(m,* 1H, C1H). δ_{C} (D₂O, 75 MHz) 19.6 (-CH₃), 52.4 (C6, C6'), 58.7 (C1), 174.4 (C5, C5'), 191.8 (C3, C3'). νₘₐₓ(neat)/cm⁻¹ 3257 (w), 2985 (w), 2931 (w), 2864 (w), 1681 (m), 1557 (s), 1442 (m), 1362 (s), 1321 (s), 1199 (s), 1052 (m), 984 (m), 826 (m), 789 (m), 745 (w), 713 (w), 659 (w), 609 (m), 483 (w), 439 (m).

### Calcium(II)N,N'-(methylmethylen)bishydantoinat (15)

N,N'-(Methylmethylen)bishydantoin **(12)** (1.0 g, 0.004 mol) wurde in H₂O_{dest}. (5 mL) suspendiert und mit Calciumhydroxid (0.3 g, 0.004 mol) versetzt. Dabei entstand eine leicht braune Suspension. Diese wurde abfiltriert und das Filtrat wurde am Rotationsverdampfer eingeengt. Eine nachfolgende Trocknung bei 50°C am Hochvakuum ergab weißes, festes Calcium(II)N,N'-(methylmethylen)bishydantoinat **(15)** (0.7 g, 57%). δ_{H} (D₂O, 300 MHz) 1.46 *(d,* 3H, J= 7 Hz, -CH₃), 3.85 *(m,* 4H, C6H₂, C6'H₂), 5.91 *(m,* 1H, C1H). δ_{C} (D₂O, 75 MHz) 19.1 (-CH3), 48.9 (C6, C6'), 62.2 (C1), 163.34 (C5, C5'), 180.2 (C3, C3'). νₘₐₓ(neat)/cm⁻¹ 3338 (m), 2041 (w), 1566 (s), 1440 (m), 1383 (s), 1312 (m), 1205 (m), 948 (w), 826 (w), 789 (w), 609 (m), 431 (w).

### Barium(II)N,N'-(methylmethylen)bishydantoinat (16)

N,N'-(Methylmethylen)bishydantoin **(12)** (1.0 g, 0.004 mol) wurde in H₂O_{dest}. (5 mL) suspendiert und mit Bariumhydroxid·Octahydrat (1.4 g, 0.004 mol) versetzt. Dabei entstand eine weiße Suspension. Diese wurde abfiltriert und das Filtrat wurde am Rotationsverdampfer eingeengt. Eine nachfolgende Trocknung bei 50°C am Hochvakuum ergab weißes, festes Barium(II)N,N'-(methylmethylen)bishydantoinat (**16**) (0.9 g, 56%). δ_{H} (D₂O, 300 MHz) 1.46 *(d,* 3H, J= 7 Hz, -CH₃), 3.85 *(m,* 4H, C6H₂, C6'H₂), 5.90 (*m,* 1H, C1H). δ_{C} (D₂O, 75 MHz) 19.1 (-CH3), 48.9 (C6, C6'), 62.1 (C1), 163.4 (C5, C5'), 180.1 (C3, C3'). νₘₐₓ(neat)/cm⁻¹ 3337 (m), 3197 (m), 3000 (w), 2939 (w), 1660 (m), 1555 (s), 1439 (m), 1384 (s), 1310 (m), 1204 (m), 1167 (m), 1074 (w), 990 (w), 947 (w), 855 (m), 828 (w), 788 (w), 748 (w), 662 (m), 607 (w), 547 (w), 431 (w).

### Bis(1,1,3,3-tetramethylguanidinium)N,N'-(methylmethylen)bishydantoinat (17)

N,N'-(Methylmethylen)bishydantoin **(12)** (1.0 g, 0.004 mol) wurde in H₂O_{dest}. (5 mL) suspendiert und mit 1,1,3,3-Tetramethylguandin (1.1 mL, 0.009 mol) versetzt. Nachdem alles gelöst war, wurde das Lösungsmittel am Rotationsverdampfer entfernt und eine nachfolgende Trocknung bei 50°C am Hochvakuum ergab Bis(1,1,3,3-tetramethylguanidinium)N,N'-(methylmethylen)bishydantoinat **(17)** (2.0 g, 98%) als klare, viskose Flüssigkeit. δ_{H} (D₂O, 300 MHz) 1.47 *(d,* 3H, J= 7 Hz, -CH₃), 2.94 (s, 24H, 8x TMG-CH₃), 3.90 *(m,* 4H, C6H₂, C6'H₂), 5.83 (*m*, 1H, C1H). δ_{C} (D₂O, 75 MHz) 19.5 (C₁-CH₃), 41.5 (8x TMG-CH₃), 52.3 (C6, C6'), 58.9 (C1), 164.1 (TMG-C1), 174.2 (C5, C5'), 191.5 (C3, C3'). νₘₐₓ(neat)/cm⁻¹ 2932 (m), 1683 (m), 1555 (s), 1447 (m), 1407 (m), 1352 (m), 1319 (m), 1195 (s), 1095 (m), 1063 (m), 1035 (m), 881 (w), 790 (m), 756 (m), 686 (m), 656 (m), 609 (m), 592 (m), 424 (m).

### Bis(tetramethylammonium)N,N'-(methylmethylen)bishydantoinat (18)

N,N'-(Methylmethylen)bishydantoin **(12)** (1.0 g, 0.004 mol) wurden in H₂O_{dest}. (5 mL) suspendiert und mit Tetramethylammoniumhydroxid (1.6 g, 0.009 mol) versetzt. Nachdem alles gelöst war, wurde das Lösungsmittel am Rotationsverdampfer entfernt und eine nachfolgende Trocknung bei 50°C am Hochvakuum ergab Bis(tetramethylammonium)N,N'-(methylmethylen)bishydantoinat **(18)** (1.22 g, 74%) als gelbliches Öl, welches sich langsam verfestigte. δ_{H} (D₂O, 300 MHz) 1.47 *(d,* 3H, J= 7 Hz, -CH₃), 3.17 (s, 24H, 8x N-CH₃), 3.90 (*m,* 4H, C6H₂, C6'H₂), 5.83 (*m,* 1H, C1H). δ_{C} (D₂O, 75 MHz) 19.1 (C₁-CH₃), 52.5 (C6, C6'), 57.9 (8x N-CH₃), 58.5 (C1), 174.8 (C5, C5'), 192.3 (C3, C3'). νₘₐₓ(neat)/cm⁻¹ 3382 (w), 3256 (w), 3022 (m), 2148 (w), 1678 (m), 1567 (s), 1490 (m), 1451 (m), 1347 (m), 1306 (m), 1197 (m), 1159 (m), 1071 (w), 1043 (w), 949 (w), 823 (w), 793 (m), 608 (m), 589 (m), 455 (m), 432 (m).

### Natrium(I)hydantoinat (19)

Hydantoin (30.0 g, 0.3 mol) wurde in H₂O_{dest}. (100 mL) suspendiert und mit Natriumhydroxid (11.9 g, 0.3 mol) versetzt und gerührt, bis sich alles gelöst hat. Das Lösungsmittel wurde am Rotationsverdampfer entfernt und eine anschließende Trocknung am Hochvakuum ergab weißes, festes und hydratisiertes Natrium(I)hydantoinat **(19)** (43.3 g, 91%). δ_{H} (DMSO-d₆/D₂O, 300 MHz) 3.42 (s, 1H, NH), 3.75 (s, 2H, CH₂). δ_{C} (DMSO-d₆/D₂O, 75 MHz) 49.5 (C5), 175.6 (C4), 188.9 (C2). νₘₐₓ(neat)/cm⁻¹ 3578 (w), 3373 (w), 3271 (m), 3154 (w), 2935 (w), 1701 (m), 1661 (m), 1508 (s), 1443 (m), 1404 (s), 1386 (s), 1311 (m), 1282 (m), 1223 (m), 1124 (w), 1090 (w), 1032 (w), 985 (w), 904 (w), 796 (m), 745 (m), 666 (m), 597 (w), 559 (m), 438 (m).

### Kalium(I)hydantoinat (20)

Hydantoin (200.0 g, 2.0 mol) wurde in H₂O_{dest}. (500 mL) suspendiert und mit Kaliumhydroxid (112,0 g, 2.0 mol) versetzt und gerührt, bis sich alles gelöst hat. Das Lösungsmittel wurde am Rotationsverdampfer entfernt und eine anschließende Trocknung am Hochvakuum ergab weißes, festes und hydratisiertes Kalium(I)hydantoinat **(20)** (301.6 g, 97%). δ_{H} (DMSO-d₆/ D₂O, 300 MHz) 3.45 (s, 1H, NH), 3.98 (s, 2H, CH₂). δ_{C} (DMSO-d₆/ D₂O, 75 MHz) 49.8 (C5), 176.1 (C4), 189.8 (C2). νₘₐₓ(neat)/cm⁻¹ 3213 (m), 3065 (w), 2973 (w), 2923 (w), 1713 (m), 1687 (m), 1574 (s), 1444 (m), 1394 (s), 1305 (m), 1218 (s), 1095 (w), 1021 (w), 985 (w), 935 (w), 894 (w), 794 (w), 745 (m), 660 (s), 588 (m), 567 (m), 452 (m).

### Calcium(II)hydantoinat (21)

Hydantoin (20.0 g, 0.2 mol) wurde in H₂O_{dest}. (50 mL) suspendiert und mit Calciumhydroxid (7.4 g, 0.1 mol) versetzt. Es bleibt eine leicht getrübte Lösung. Diese wurde abfiltriert und am Rotationsverdampfer eingeengt. Eine anschließende Trocknung am Hochvakuum ergab festes, weißes und hydratisiertes Calcium(II)hydantoinat **(21)** (25.5 g, 93%). δ_{H} (D₂O, 300 MHz) 3.66 (*s*, 4H, CH₂). δ_{C} (c, 75 MHz) 46.5 (C5), 164.0 (C4), 180.9 (C2). νₘₐₓ(neat)/cm⁻¹ 3641 (w), 3392 (m), 3212 (w), 2939 (w), 1637 (s), 1571 (s), 1436 (s), 1395 (s), 1378 (m), 1322 (s), 1210 (m), 1157 (w), 1048 (m), 930 (w), 912 (w), 875 (m), 776 (m), 732 (w), 674 (m), 583 (s), 552 (s), 445 (m).

### Barium(II)hydantoinat (22)

Hydantoin (20.0 g, 0.2 mol) wurde mit H₂Odest (10 ml) suspendiert. Nach der Zugabe von Bariumhydroxid·Octahydrat (31.5 g, 0.1 mol) entstand eine kleisterähnliche weiße Substanz. Durch Filtration wurde die Lösung abgetrennt und bei vermindertem Druck wurde das Wasser abdestilliert. Trocknung im Vakuum über Phosphorpentoxid ergab Barium(II)hydantoin **(22)** (25.3 g, 76%). νₘₐₓ(neat)/cm⁻¹ 3188 (m), 1673 (m), 1574 (s), 1442 (m), 1390 (s), 1301 (m), 1218 (m), 1103 (w), 1022 (w), 992 (w), 907 (w), 794 (w), 736 (m), 661 (m), 639 (m), 600 (w), 577 (m), 546 (m), 433 (m).

### 1,1,3,3-Tetramethylguanidiniumhydantoinat (23)

Hydantoin (2.0 g, 0.02 mol) wurde in H₂O_{dest}. (10 mL) suspendiert und mit 1,1,3,3-Tetramethylguanidin (2.5 mL, 0.02 mol) versetzt. Dabei löst sich der Feststoff sehr rasch. Das Lösungsmittel wurde am Rotationsverdampfer entfernt und eine anschließende Trocknung am Hochvakuum ergab weiß-kristallines 1,1,3,3-Tetramethylguanidiniumhydantoinat (**23**) (4.1 g, 95%). δ_{H} (D₂O, 300 MHz) 2.95 (s, 12 H, TMG-CH₃), 3.87 (s, 2 H,CH₂). δ_{C} (D₂O, 75 MHz) 41.5 (TMG-CH3), 52.1 (C5), 164,0 (C4), 193.4 (C2), 177.8 (TMG-C1). νₘₐₓ(neat)/cm⁻¹ 3286 (m), 3064 (m), 2935 (m), 2869 (m), 2777 (m), 1693 (m), 1557 (s), 1455 (m), 1409 (m), 1370 (m), 1311 (w), 1275 (m), 1212 (m), 1169 (m), 1110 (m), 1064 (m), 1035 (m), 877 (m), 792 (m), 738 (m), 687 (m), 654 (m), 597 (m), 558 (m), 430 (m).

### Tetramethylammoniumhydantoinat (24)

Hydantoin (2.0 g, 0.02 mol) wurde in H₂O_{dest}. (10 mL) suspendiert und mit Tetramethylammoniumhydroxid·Pentahydrat (3.6 g, 0.02 mol) versetzt. Dabei löst sich der Feststoff sehr rasch. Das Lösungsmittel wurde am Rotationsverdampfer entfernt und eine anschließende Trocknung am Hochvakuum ergab weiß-kristallines Tetramethylammoniumhydantoinat **(24)** (3.2 g, 92%). δ_{H} (D₂O, 300 MHz) 3.18 (*s*, 12H, 4x N-CH₃), 3.86 (s, 2H, CH₂). δ_{C} (D₂O, 75 MHz) 52.2 (C5), 57.9 (N-CH₃), 178.8 (C4), 194.8 (C2). νₘₐₓ(neat)/cm⁻¹ 3256 (m), 3021 (m), 1682 (w), 1568 (s), 1487 (m), 1460 (m), 1391 (m), 1357 (m), 1281 (m), 1211 (s), 1091 (m), 1011 (w), 950 (s), 887 (w), 800 (m), 739 (m), 690 (m), 649 (s), 593 (m), 580 (m), 456 (w), 428 (m).

### Tetrakis(hydroxymethyl)phosphoniumhydantoinat (25)

Barium(II)hydantoinat **(23)** (25.3 g 0.08 mol) wurde in 200 mL H₂O_{dest}. suspendiert und mit Bis[tetrakis(hydroxymethyl)phosphonium]sulfat (30.1 mL, 0.08 mol) versetzt. Daraufhin bildete sich ein feiner, weißer Niederschlag, der abfiltriert wurde, und das Lösungsmittel wurde am Rotationsverdampfer entfernt. Eine anschließende Trocknung am Hochvakuum bei 50°C ergab Tetrakis(hydroxymethyl)phosphoniumhydantoinat in einer Mischung mit nicht ungesetzten Edukten **(25)** (28.7 g, 74%). νₘₐₓ(neat)/cm⁻¹ 3391 (m), 3261 (m), 2977 (w), 2935 (w), 2898 (w), 1782 (m), 1693 (s), 1461 (m), 1442 (m), 1379 (m), 1339 (m), 1296 (m), 1208 (m), 1154 (m), 1083 (m), 1039 (s), 928 (m), 779 (m), 761 (m), 716 (m), 679 (m), 638 (s), 580 (s), 479 (m).

### Natrium(I)allantoinat (26)

Allantoin (2.0 g, 0.01 mol) wurde in H₂O_{dest}. (10 mL) suspendiert und mit Natriumhydroxid (0.5 g, 0.01 mol) versetzt. Sobald alles gelöst war, wurde das Lösungsmittel am Rotationsverdampfer entfernt und eine anschließende Trocknung am Hochvakuum bei 50°C ergab weißes, festes Natrium(I)allantoinat **(26)** (2.0 g, 89%). δ_{H} (D₂O, 300 MHz) 5.27 (*s*, 1H, C5H). δ_{C} (D₂O, 75 MHz) 62.9 (C5), 163.2 (C7), 177.4 (C4), 192.5 (C2). νₘₐₓ(neat)/cm⁻¹ 3297 (m), 3208 n(m), 1574 (s), 1532 (s), 1382 (s), 1304 (m), 1197 (m), 1162 (m), 1038 (w), 790 (w), 720 (w), 601 (w), 513 (w), 437 (w).

### Kalium(I)allantoinat (27)

Allantoin (2.0 g, 0.01 mol) wurde in H₂O_{dest}. (10 mL) suspendiert und mit Kaliumhydroxid (0.8 g, 0.01 mol) versetzt. Sobald alles gelöst war, wurde das Lösungsmittel am Rotationsverdampfer entfernt und eine anschließende Trocknung am Hochvakuum bei 50°C ergab weißes, festes Kalium(I)allantoinat **(27)** (1.3 g, 52%). δ_{H} (D₂O, 300 MHz) 5.35 (*s*, 1H, C5H). δ_{C} (D₂O, 75 MHz) 67.2 (C5), 163.2 (C7), 176.3 (C4), 191.7 (C2). νₘₐₓ(neat)/cm⁻¹ 3266 (m), 1661 (m), 1574 (s), 1536 (s), 1374 (m), 1285 (m), 1163 (m), 1034 (m), 785 (m),720 (w), 647 (w), 608 (w), 512 (w), 436 (m).

### Calcium(II)allantoinat (28)

Allantoin (2.0 g, 0.013 mol) wurde in H₂O_{dest}. (10 mL) suspendiert und mit Calciumhydroxid (0.5 g, 0.006 mol) versetzt. Es entstand eine leicht bräunliche Suspension, die abfiltriert wurde, und das Filtrat wurde am Rotationsverdampfer eingeengt. Eine anschließende Trocknung am Hochvakuum bei 50°C ergab weißes, festes Calcium(II)allantoinat (**28**) (1.9 g, 83%). δ_{H} (D₂O, 300 MHz) 5.30 (s, 1H, C5H). δ_{C} (D₂O, 75 MHz) 62.9 (C5), 163.2 (C7), 177.5 (C4), 191.6 (C2). νₘₐₓ(neat)/cm⁻¹ 3307 (m), 3207 (m), 1575 (s), 1520 (s), 1385 (s), 1156 (m), 779 (m).

### Barium(II)allantoinat (29)

Allantoin (2.0 g, 0.013 mol) wurde in H₂O_{dest}. (10 mL) suspendiert und mit Bariumhydroxid·Octahydrat (2.0 g, 0.006 mol) versetzt. Es entstand eine weiße Suspension, die abfiltriert wurde, und das Filtrat wurde am Rotationsverdampfer eingeengt. Eine anschließende Trocknung am Hochvakuum bei 50°C ergab weißes, festes Barium(II)allantoinat (**29**) (1.8 g, 62%). δ_{H} (D₂O, 300 MHz) 5.30 (s, 1H, CH), 5.37 (s, 1H, C5'H). δ_{C} (D₂O, 75 MHz) 67.2 (C5), 163.2 (C7), 177.3 (C4), 191.6 (C2). νₘₐₓ(neat)/cm⁻¹ 3285 (m), 3204 (m), 1651 (m), 1569 (s), 1532 (s), 1380 (m), 1291 (m), 1159 (m), 1039 (w), 782 (m).

### 1,1,3,3-Tetramethylguanidiniumallantoinat (30)

Allantoin (2.0 g, 0.01 mol) wurde in H₂O_{dest}. (10 mL) suspendiert und mit 1,1,3,3-Tetramethylguanidin (1.6 mL, 0.01 mol) versetzt. Nachdem sich alles gelöst hatte, wurde das Lösungsmittel am Rotationsverdampfer entfernt und eine anschließende Trocknung am Hochvakuum bei 50°C ergab weißes, festes 1,1,3,3-Tetramethylguanidiniumallantoinat (**30**) (2.9 g, 84%). δ_{H} (D₂O, 300 MHz) 2.95 (s, 12H, 4x TMG-CH₃) 5.35 (s, 1H, C5H). δ_{C} (D₂O, 75 MHz) 41.5 (4x N-CH₃), 67.2 (C5), 163.2 (C7), 164.1 (TMG-C1), 177.0 (C4), 192.2 (C2). νₘₐₓ (neat)/cm⁻¹ 3179 (m), 2938 (m), 2819 (m), 1659 (m), 1557 (s), 1407 (m), 1371 (m), 1311 (m), 1283 (m), 1167 (m), 1092 (m), 1061 (m), 1034 (m), 916 (w), 878 (w), 794 (m), 702 (m), 643 (m), 612 (m), 515 (m), 448 (m).

### Tetramethylammoniumallantoinat (31)

Allantoin (2.0 g, 0.01 mol) wurde in H₂O_{dest}. (10 mL) suspendiert und mit Tetramethylammoniumhydroxid·Pentahydrat (2.3 g, 0.01 mol) versetzt. Nachdem sich alles gelöst hatte, wurde das Lösungsmittel am Rotationsverdampfer entfernt und eine anschließende Trocknung am Hochvakuum bei 50°C ergab weißes, festes und hydratisiertes Tetramethylammoniumallantoinat **(31)** (3.3 g, 98%). δ_{H} (D₂O, 300 MHz) 3.17 (s, 12H, 4x N-CH₃) 5.30 (*m*, 1H, C5H). δ_{C} (D₂O, 75 MHz) 57.9 (4x N-CH₃), 67.2 (C5), 163.2 (C7), 177.1 (C4), 192.2 (C4). νₘₐₓ(neat)/cm⁻¹ 3432 (m), 3343 (m), 3179 (m), 2791 (w), 1722 (w), 1663 (m), 1613 (s), 1540 (m), 1483 (s), 1448 (w), 1411 (w), 1377 (m), 1280 (m), 1193 (m), 1153 (m), 1073 (m), 1039 (m), 947 (m), 917 (w), 800 (m), 722 (m), 669 (m), 646 (m), 623 (s), 569 (m), 531 (m), 453 (m), 405 (m).

### Dihydroxyaluminiumallantoinat (32) (DE 3034506)

Zu AlCl₃ (2.0 g, 0.02 mol) wurde bei 0°C H₂O_{dest}. (10 ml) zugetropft (10 min). Anschließend wurde Natrium(I)allantoinat **(26)** (2.7 g, 0.02 mol) in H₂O_{dest}. (5 ml) zugetropft. Dabei bildet sich sofort ein weißer Niederschlag. Der Niederschlag wurde abgenutscht und mit H₂O_{dest}. nachgewaschen. Trocknung am Hochvakuum ergab weißes, pulvriges Dihydroxyaluminiumallantoinat (**32**).(1.0 g, 35%). νₘₐₓ(neat)/cm⁻¹ 3434 (m), 3338 (m), 3187 (m), 3051 (m), 2760 (w), 1777 (m), 1701 (s), 1658 (s), 1601 (m), 1524 (s), 1428 (m), 1397 (m), 1359 (m), 1324 (m), 1281 (m), 1180 (s), 1059 (w), 1014 (m), 968 (w), 814 (m), 777 (w), 759 (m), 705 (m), 670 (w), 629 (m), 557 (m), 502 (m), 450 (m), 434 (m).

### Methylenbis(barbitursäure) (33)^{[1]}

Zu Barbitursäure (11.3 g, 0.09 mol) in H₂O_{dest}. (435 ml) wurde 1 M Kalilauge (43.5 ml, 0.04 mol) sowie Diethylamin (0.5 g, 0.01 mol) als Katalysator gegeben. Nach dem Erwärmen auf 80°C wurde 35%ige Formaldehydlösung (3.7 g, 0.04 mol) zugetropft. Anschließend wurde 1 h bei 80°C gerührt und danach der pH-Wert mit 37%iger Salzsäure auf 1-2 eingestellt (Verbrauch: 10 ml). Trocknung im Vakuum über Phosphorpentoxid ergab 3,3'-Methylenbis(barbitursäure) **(33)** (11.5 g, 99%). νₘₐₓ (neat)/cm⁻¹ 3119 (w), 2943 (w), 2804 (w), 1645 (s), 1494 (s), 1406 (m), 1367 (m), 1278 (w), 1214 (w), 1187 (w), 1139 (w), 1060 (m), 871 (m), 831 (m), 763 (m), 745 (m), 674 (w), 642 (w), 585 (m), 557 (m), 476 (w), 442 (m) cm⁻¹.
[1] Gynsling, H.; Schwarzenbach, G.: Metallindikatoren II. Beziehungen zwischen Struktur und Komplexbildungsvermögen bei Verwandten des Murexids. Helvetica Chimica Acta. 10/2004; 32(5):1484 - 1504.

## Patentansprüche

1. Verbindung der Formel 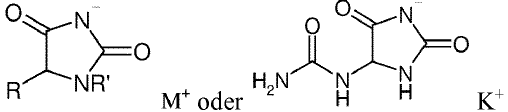 oder ein Hydrat davon,
wobei
R ausgewählt ist aus der Gruppe, bestehend aus:
H, C₁-C₅-Alkyl und 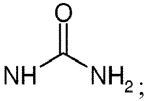
R' ausgewählt ist aus der Gruppe, bestehend aus: 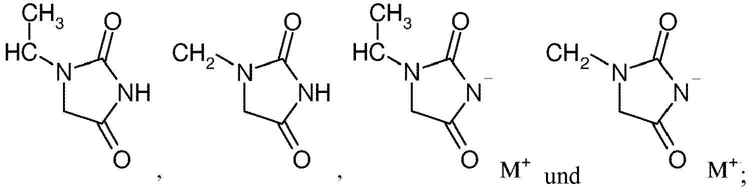 und
M⁺ entweder K⁺ oder Na⁺ ist.

2. Verbindung nach Anspruch 1, ausgewählt aus: 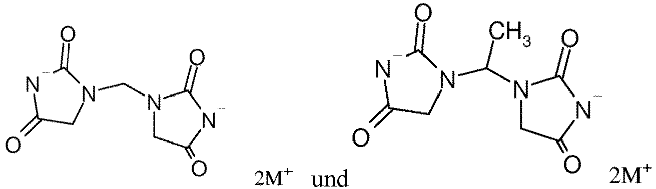 und den Hydraten davon.

3. Stoffgemisch, umfassend:
(i) eine erste Verbindung einer Formel, ausgewählt aus der Gruppe, bestehend aus: 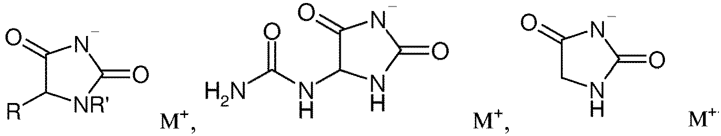 und den Hydraten davon, wobei R und R' wie in Anspruch 1 definiert sind und M⁺ = K⁺; und
(ii) eine zweite Verbindung einer Formel, ausgewählt aus der Gruppe, bestehend aus: 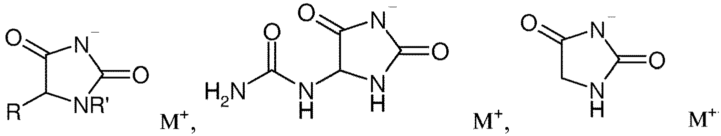 und den Hydraten davon, wobei R und R' wie in Anspruch 1 definiert sind und M⁺ = Na⁺.

4. Stoffgemisch nach Anspruch 3, wobei die erste Verbindung Kaliumhydantoin und die zweite Verbindung Natriumhydantoin ist.

5. Intumeszenzbeschichtung, umfassend eine Verbindung nach Anspruch 1 oder Anspruch 2 oder ein Stoffgemisch nach Anspruch 3 oder Anspruch 4.

6. Intumeszenzbeschichtung, bestehend aus:
(a) einer Verbindung einer Formel, ausgewählt aus der Gruppe, bestehend aus: 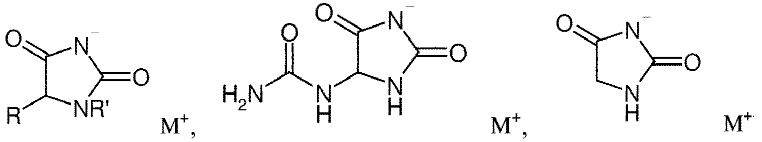 und den Hydraten davon, wobei R, R' und M⁺ wie in Anspruch 1 definiert sind, oder Gemischen der Verbindungen; und
(b) Wasser.

7. Verfahren zum Aufbringen einer Intumeszenzbeschichtung auf ein Substrat, **dadurch gekennzeichnet, dass** eine wässrige Lösung einer Verbindung einer Formel, ausgewählt aus der Gruppe, bestehend aus: 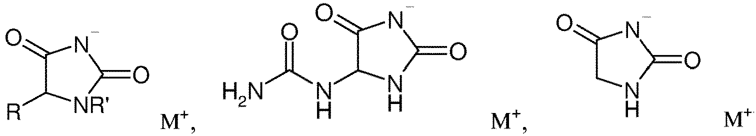 und den Hydraten davon, wobei R, R' und M⁺ wie in Anspruch 1 definiert sind, oder eines Stoffgemischs nach Anspruch 3 oder Anspruch 4 auf das Substrat aufgebracht wird.

8. Verwendung einer Verbindung einer Formel, ausgewählt aus der Gruppe, bestehend aus: 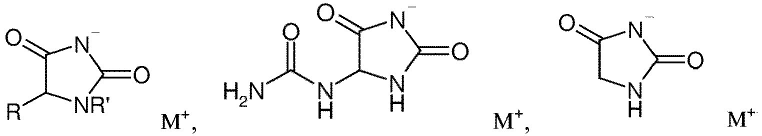 und den Hydraten davon, wobei R, R' und M⁺ wie in Anspruch 1 definiert sind, oder eines Stoffgemischs nach Anspruch 3 oder Anspruch 4 als Intumeszenzbeschichtung.

## Claims

1. A compound having the formula 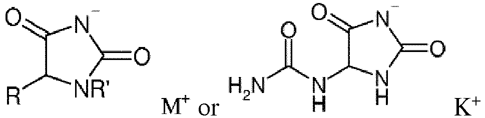 or a hydrate thereof,
wherein
R is selected from the group consisting of:
H, C₁-C₅ alkyl, and 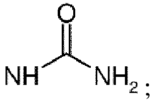
R' is selected from the group consisting of: 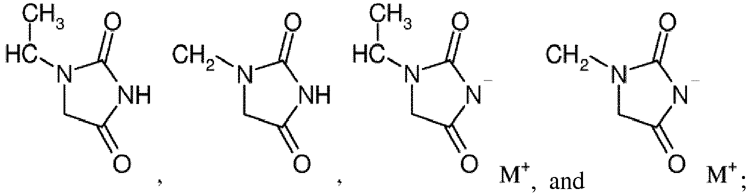 and
M⁺ is either K⁺ or Na⁺.

2. The compound of claim 1, selected from: 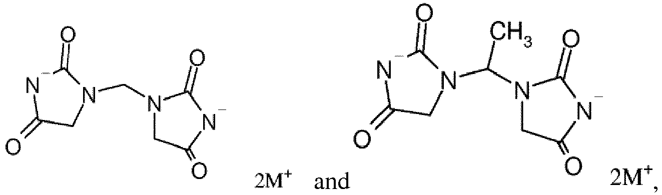 and the hydrates thereof.

3. A mixture of substances, comprising:
(i) a first compound having a formula selected from the group consisting of: 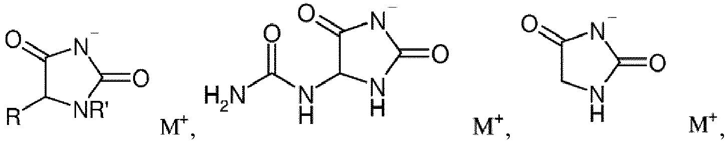 and the hydrates thereof, wherein R and R' are as defined in claim 1 and M⁺ = K⁺; and
(ii) a second compound having a formula selected from the group consisting of: 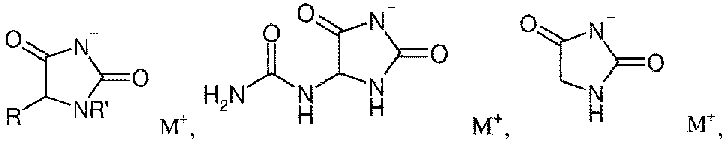 and the hydrates thereof, wherein R and R' are as defined in claim 1 and M⁺ = Na⁺.

4. The mixture of substances of claim 3, wherein the first compound is potassium hydantoin and the second compound is sodium hydantoin.

5. An intumescent coating comprising a compound of claim 1 or claim 2 or a mixture of substances of claim 3 or claim 4.

6. An intumescent coating consisting of:
(a) a first compound having a formula selected from the group consisting of: 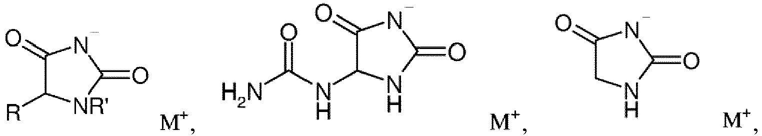 and the hydrates thereof, wherein R, R' and M⁺ are as defined in claim 1, or mixtures of the compounds; and
(b) water.

7. A method of applying an intumescent coating onto a substrate, **characterized in that** an aqueous solution of a compound having a formula selected from the group consisting of: 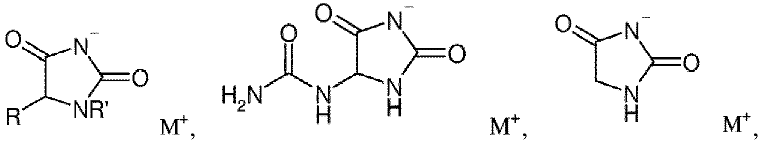 and the hydrates thereof, wherein R, R' and M⁺ are as defined in claim 1, or of a mixture of substances of claim 3 or claim 4 is applied onto the substrate.

8. Use of a compound having a formula selected from the group consisting of: 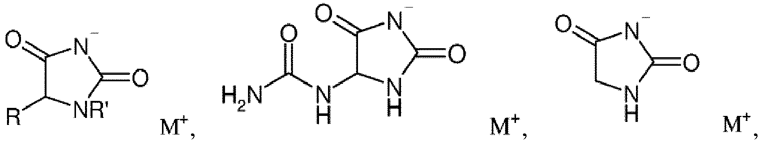 and the hydrates thereof, wherein R, R' and M⁺ are as defined in claim 1, or of a mixture of substances of claim 3 or claim 4 as intumescent coating.

## Revendications

1. Composé de formule 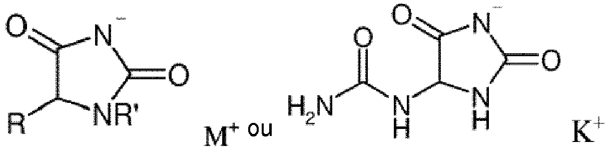 ou hydrate de celui-ci,
dans lequel
R est choisi dans le groupe consistant en : H, les groupes alkyles en C₁-C₅ et 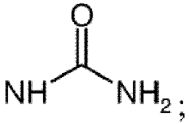
R' est choisi dans le groupe consistant en : 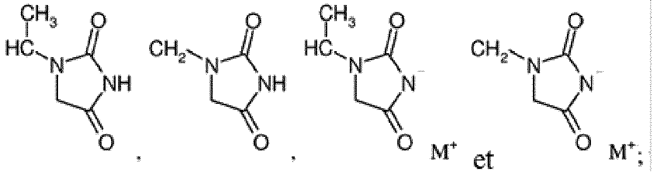 et
M⁺ est K⁺ ou Na⁺.

2. Composé selon la revendication 1, choisi parmi : 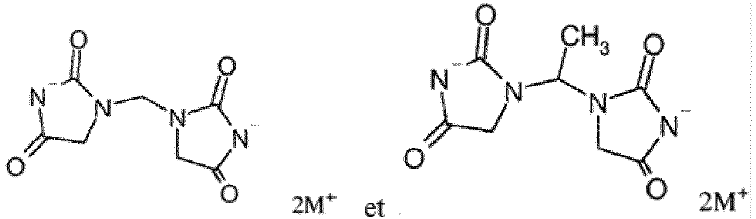 et les hydrates de ceux-ci.

3. Mélange de substances, comprenant :
(i) un premier composé ayant une formule choisie dans le groupe consistant en : 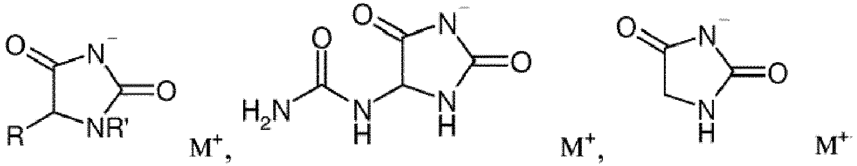 et les hydrates de ceux-ci, R et R' étant tels que définis dans la revendication 1, et M⁺ = K⁺ ; et
(ii) un second composé ayant une formule choisie dans le groupe consistant en : 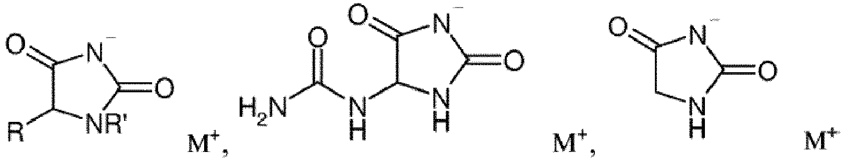 et les hydrates de ceux-ci, où R et R' sont tels que définis dans la revendication 1, et M⁺ = Na+.

4. Mélange de substances selon la revendication 3, dans lequel le premier composé est l'hydantoïne de potassium et le second composé est l'hydantoïne de sodium.

5. Revêtement intumescent, comprenant un composé selon la revendication 1 ou la revendication 2, ou un mélange de substances selon la revendication 3 ou la revendication 4.

6. Revêtement intumescent, consistant en :
(a) un composé ayant une formule choisie dans le groupe consistant en : 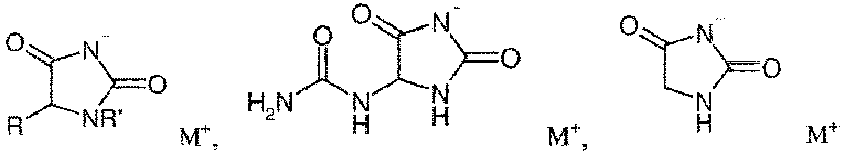 et les hydrates de ceux-ci, R, R' et M⁺ étant tels que définis dans la revendication 1, ou les mélanges des composés ; et
(b) de l'eau.

7. Procédé pour appliquer un revêtement intumescent sur un substrat, **caractérisé en ce qu'**on applique sur le substrat une solution aqueuse d'un composé ayant une formule choisie dans le groupe consistant en : 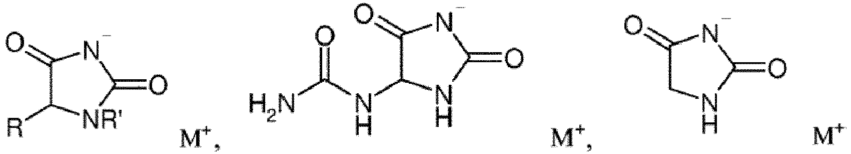 et les hydrates de ceux-ci, R, R' et M⁺ étant tels que définis dans la revendication 1, ou un mélange de substances selon la revendication 3 ou la revendication 4.

8. Utilisation d'un composé ayant une formule choisie dans le groupe consistant en : 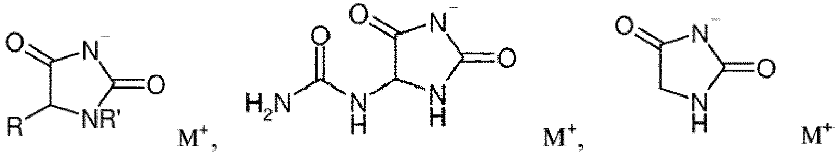 et les hydrates de ceux-ci, R, R' et M⁺ étant tels que définis dans la revendication 1, ou d'un mélange de substances selon la revendication 3 ou 4, en tant que revêtement intumescent.
